(19) **Europäisches Patentamt**

**European Patent Office**

**Office européen des brevets**

(11) **EP 1 215 207 B1**

(12) **EUROPÄISCHE PATENTSCHRIFT**

(45) Veröffentlichungstag und Bekanntmachung des Hinweises auf die Patenterteilung:
**28.07.2004 Patentblatt 2004/31**

(51) Int Cl.[7]: **C07D 277/04**, C07D 295/185, A61K 31/426, A61K 31/40, A61P 3/10

(21) Anmeldenummer: **02005352.6**

(22) Anmeldetag: **28.05.1999**

(54) **Organische Salze von Isoleucyl-Thiazolidin und -Pyrrolidin und ihre Verwendung als Dipeptidylpeptidase-Inhibitoren**

Organic Salts of isoleucyl-thiazolidine and -pyrrolidine and their use as dipeptidylpeptidase inhibitors

Sels organiques d'isoleucyl-thiazolidine et -pyrrolidine et leur utilisation comme inhibiteurs de dipeptidylpeptidase

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **28.05.1998 DE 19823831**

(43) Veröffentlichungstag der Anmeldung:
**19.06.2002 Patentblatt 2002/25**

(62) Dokumentnummer(n) der früheren Anmeldung(en) nach Art. 76 EPÜ:
**99926464.1 / 1 082 314**

(73) Patentinhaber: **Probiodrug AG**
**06120 Halle/Saale (DE)**

(72) Erfinder:
• **Demuth, Hans-Ulrich**
**06114 Halle (DE)**
• **Glund, Konrad**
**06122 Halle (DE)**
• **Schlenzig, Dagmar**
**06114 Halle (DE)**
• **Kruber, Susanne**
**06114 Halle (DE)**

(74) Vertreter:
**Forstmeyer, Dietmar, Dr. rer. nat., Dipl.-Chem. et al**
**Boeters & Bauer,**
**Bereiteranger 15**
**81541 München (DE)**

(56) Entgegenhaltungen:
**WO-A-95/15309          WO-A-97/40832**
**DD-A- 296 075**

• **ASHWORTH D M ET AL: "2-Cyanopyrrolidides as potent, stable inhibitors of dipeptidyl peptidase IV" BIOORGANIC & MEDICINAL CHEMISTRY LETTERS, Bd. 6, Nr. 10, 21. Mai 1996 (1996-05-21), Seiten 1163-1166, XP004134889**
• **DATABASE CAPLUS [Online] CHEMICAL ABSTRACTS SERVICE, COLUMBUS, OHIO, US; Database accession no. 1973:442838 XP002196289 & JP 48 032802 A (YAMADA S ET AL) 2. Mai 1973 (1973-05-02)**

Bemerkungen:
Die Akte enthält technische Angaben, die nach dem Eingang der Anmeldung eingereicht wurden und die nicht in dieser Patentschrift enthalten sind.

**Beschreibung**

Die vorliegende Erfindung betrifft Dipeptidverbindungen bzw. zu Dipeptidverbindungen analoge Verbindungen, die aus einer Aminosäure und einer Thiazolidin- oder Pyrrolidingruppe gebildet werden, und deren Salze, im weiteren Dipeptidverbindungen genannt, und die Verwendung der Verbindungen zur Behandlung von beeinträchtigter Glukosetoleranz, Glukosurie, Hyperlipidämie, metabolischen Azidosen, Diabetes mellitus, diabetischer Neuropathie und Nephropathie sowie von durch Diabetes mellitus verursachten Folgeerkrankungen von Säugern.

Die Erfindung betrifft also auch ein einfaches Verfahren zur Senkung der Blutzuckerkonzentration von Säugern mit Hilfe von Dipeptidverbindungen als aktivitätsmindernde Effektoren (Substraten, Pseudosubstraten, Inhibitoren, Bindungsproteinen, Antikörpern u. a.) für Enzyme mit vergleichbarer oder identischer Aktivität zur enzymatischen Aktivität des Enzyms Dipeptidyl Peptidase IV.

DP IV- bzw. DP IV-analoge Aktivität (z. B. besitzt die cytosolische DP II eine der DP IV nahezu identische Substratspezifität) kommt im Blutkreislauf vor, wo sie hochspezifisch Dipeptide vom N-Terminus biologisch aktiver Peptide abspaltet, wenn Prolin oder Alanin die benachbarten Reste der N-terminalen Aminosäure in deren Sequenz darstellen.

Die Glukose-abhängigen insulinotropen Polypeptide: Gastric Inhibitory Polypeptide 1-2 ($GIP_{1-42}$) und Glucagon-Like Peptide Amide-1 7-36 ($GLP-1_{7-36}$), also Hormone, die die Glukoseinduzierte Insulinsekretion des Pankreas stimulieren (auch Incretine genannt), sind Substrate der DP IV, da diese von den N-terminalen Sequenzen dieser Peptide die Dipeptide Tyrosinyl-Alanin bzw. Histidyl-Alanin in vitro und in vivo abspalten kann.

Die Reduktion derartiger DP IV- bzw. DP IV-analoger Enzymaktivität zur Spaltung solcher Substrate *in vivo* kann dazu dienen, unerwünschte Enzymaktivität unter Laborbedingungen wie auch bei pathologischen Zuständen von Säuger-Organismen wirksam zu unterdrücken. Z.B. basiert *Diabetes mellitus* Typ II (auch Altersdiabetes) auf einer verminderten Insulinsekretion bzw. Störungen in der Rezeptorfunktion, die u.a. in proteolytisch bedingten Konzentrationsanomalien der Incretine begründet sind.

Hyperglykämie und damit verbundene Ursachen bzw. Folgeerscheinungen (auch *Diabetes mellitus)* werden nach gegenwärtigem Stand der Technik durch die Verabreichung von Insulin (z.B. von aus Rinderpankreas isoliertem oder auch gentechnisch gewonnenem Material) an erkrankte Organismen in verschiedenen Darreichungsformen behandelt. Alle bisher bekannten, wie auch modernere Verfahren, zeichnen sich durch hohen Materialaufwand, hohe Kosten und oft durch entscheidende Beeinträchtigungen der Lebensqualität der Patienten aus. Die klassische Methode (tägliche *i.v.* Insulin-Injektion, üblich seit den dreißiger Jahren) behandelt die akuten Krankheitssymptome, führt aber nach längerer Anwendung u. a. zu schweren Gefäßveränderungen (Arteriosklerose) und Nervenschädigungen.

Neuerdings wird die Installation subkutaner Depot-Implantate (die Insulinabgabe erfolgt dosiert, und die täglichen Injektionen entfallen) sowie die Implantation (Transplantation) intakter Langerhansscher Zellen in die funktionsgestörte Pankreasdrüse oder andere Organe und Gewebe vorgeschlagen. Derartige Transplantationen sind technisch aufwendig. Weiterhin stellen sie einen risikobehafteten chirurgischen Eingriff in den Empfängerorganismus dar und verlangen auch bei Zellverpflanzungen nach Methoden zur Suppression bzw. der Umgehung des Immunsystems.

Die Verwendung von Alanyl-Pyrrolidid und Isoleucyl-Thiazolidid als Inhibitoren von DP IV bzw. von zu DP IV analoger Enzymaktivität ist bereits aus der PCT/DE 97/00820 und die Verwendung von Isoleucyl-Pyrrolid und Isoleucyl-Thiazolidid-Hydrochlorid bereits aus der DD 296 075 bekannt. Bei dem in diesem Stand der Technik eingesetzten Isoleucyl-Thiazolidid handelt es sich um natürliches, also L-threo-Isoleucyl-Thiazolidid: Zum Prioritätsdatum und noch am Anmeldetag der beiden Druckschriften stand nur diese, die natürliche Form von Isoleucyl-Thiazolidid zur Verfügung.

Ashworth et al. (Bioorganic & Medical Chemistry Letters, Vol. 6, No. 10, pp. 1163-1166, 1996) beschreiben neue Dipeptidylanaloga als potente stabile Inhibitoren der Dipeptidyl Peptidase IV und deren kinetische Charakterisierung.

Es ist festgestellt worden, daß diese Verbindungen, insbesondere L-threo-Isoleucyl-Thiazolidid, gute Effektoren für DP IV und DP IV analoge Enzymaktivitäten sind. Bei der Verwendung dieser Verbindung können bei einigen Patienten bzw. Krankheitsformen jedoch gewisse Probleme auftreten:

Je nach Symptomen und Schwere z.B. von Diabetes mellitus, wäre es z.B. wünschenswert, Effektoren zur Verfügung zu haben, die eine andere Wirkung als die bekannten Verbindungen aufweisen: So ist bekannt, daß Diabetes-mellitus-Patienten individuell "eingestellt" werden müssen, um eine optimale Behandlung ihrer Krankheit zu ermöglichen. So sollte bei einigen Fällen z.B. eine verringerte Aktivität von DP IV Effektoren ausreichen. Auch könnte eine zu hohe Inhibitor Aktivität und die permanen-te Verabreichung desselben Medikaments insbesondere wegen der lebenslangen Dauer der Behandlung unerwünschte Nebenwirkungen zur Folge haben. Weiter könnte es auch wünschenwert sein, gewisse Transporteigenschaften zur Erhöhung der Resorptionsgeschwindigkeit der Effektoren in vivo zu verbessern.

Aufgabe der Erfindung ist es daher, neue (insbesondere aktivitätsmindernde) Effektoren zur Behandlung von z.B. beeinträchtigter.Glukosetoleranz, Glukosurie, Hyperlipidämie, metabolischen Azidosen, Diabetes mellitus, diabetischer Neuropathie und Nephropathie sowie von durch Diabetes mellitus verursachten Folgeerkrankungen von Säugern und ein einfaches Verfahren zur Behandlung dieser Krankheiten bereitzustellen.

Diese Aufgabe wird erfindungsgemäß durch die Bereitstellung von organischen Salze von Dipeptidverbindungen bzw. Analoga von Dipeptiden, die aus einer Aminosäure und einer Thiazolidin- oder Pyrrolidingruppe gebildet werden nämlich organischen Salze von L-threo-Isoleucyl Thiazolidin und von L-threo-Isoleucyl Pyrrolidin, gelöst.

Bei der - vorzugsweise oralen - Verabreichung dieser Effektoren an einen Säugerorganismus werden die endogenen (oder zusätzlich exogen verabreichten) insulinotropen Peptide $GIP_{1-42}$ und $GLP-1_{7-36}$ (o.a. $GLP-1_{7-37}$ oder deren Analoga) durch DP IVoder DP IV-ähnliche Enzyme vermindert abgebaut und damit die Konzentrationsabnahme dieser Peptidhormone bzw. ihrer Analoga verringert bzw. verzögert. Der Erfindung liegt also der Befund zugrunde, daß eine Reduktion der im Blutkreislauf agierenden DP IV- oder DP IV-ähnlichen enzymatischen Aktivität zur Beeinflussung des Blutzuckerspiegels führt. Es wurde gefunden, daß

1. die Verminderung von DP IV- bzw. DP IV-analoger Aktivität zu relativer Stabilitätserhöhung der Glukosestimulierten, oder extern zugeführten Incretine (oder deren Analoga) führt, d.h. durch Applikation von Effektoren der DP IV bzw. DP IV-anloger Proteine der Incretin-Abbau im Blut kontrolliert werden kann;

2. die erhöhte biologische Abbaustabilität der Incretine (oder ihrer Analoga) eine Wirkungsveränderung endogenen Insulins zur Folge hat;

3. die durch Reduktion der DP IV- bzw. DP IV-analogen enzymatischen Aktivität im Blut erzielte Stabilitätserhöhung der Incretine in einer nachfolgenden Veränderung der Glukoseinduzierten Insulinwirkung resultiert und damit zu einer mittels DP IV-Effektoren kontrollierbaren Modulierung des Blut-Glukosespiegels führt.

Die möglichst orale Applikation der erfindungsgemäßen hochaffinen, niedermolekularen Enzyminhibitoren ist eine kostengünstigere Alternative z.B. zu invasiven chirurgischen Techniken bei der Behandlung pathologischer Erscheinungen. Durch chemisches Design von Stabilitäts-, Transport- und Clearance-Eigenschaften kann deren Wirkungsweise modifiziert und auf individuelle Eigenschaften abgestimmt werden.

Wie vorstehend erwähnt, kann es z.B. bei der Dauerbehandlung von Diabetes mellitus erforderlich sein, Effektoren mit einer definierten Aktivität zur Verfügung zu stellen, mit denen individuelle Bedürfnisse von Patienten erfüllt bzw. Symptome behandelt werden können. Die erfindungsgemäßen Dipeptidverbindungen weisen daher bei einer Konzentration (der Dipeptidverbindungen) von 10 μM, insbesondere bei den in Tabelle 1 angegebenen Bedingungen, eine Aktivitätsminderung von Dipeptidyl Peptidase IV bzw. DP IV-analoger Enzymaktivitäten von mindestens 10, bevorzugt von mindestens 40 % auf. Häufig ist auch eine Aktivitätsminderung von mindestens 60 % bzw. mindestens 70 % erforderlich. Bevorzugte Effektoren können auch eine Aktivitätsminderung von maximal 20 % bzw. 30 % aufweisen. Weiterhin sind die Transporteigenschaften der vorliegenden Verbindungen, insbesondere durch den Peptidtransporter Pep T1 deutlich verbessert.

Die organische Salze der erfindungsgemäßen Dipeptidverbindungen sind z.B. Salze wie Acetate, Succinate, Tartrate oder Fumarate Besonders bevorzugt werden die Fumarate, die eine hervorragende Wirkung bei einer überraschend hohen Stabilität gegenüber Hydrolyse aufweisen und wesentlich weniger löslich sind als die Hydrochloride. Diese Eigenschaften sind auch bei der Galenik von Vorteil.

Ferner werden die organische Salze von L-threo Isoleucyl Pyrrolidid insbesondere die Fumarsalze bevorzugt.

Die Salze der Dipeptidverbindungen können in einem molaren Verhältnis von Dipeptid(analogon)komponente zu Salzkomponente von 1 : 1 oder 2 : 1 vorliegen. Ein derartiges Salz ist z.B. $(Ile-Thia)_2$ Fumarsäure.

Besonders bevorzugte Salze sind die Fumarsalze von L-threo-Isoleucyl Thiazolidid.

Die Erfindung betrifft somit Effektoren der Dipeptidyl Peptidase IV (DP IV)- bzw. DP IV-analoger Enzymaktivität und deren Verwendung zur Senkung des Blutzuckerspiegels unter die für Hyperglykämie charakteristische Glukosekonzentration im Serum eines Säuger-Organismus. Insbesondere betrifft die Erfindung die Verwendung der erfindungsgemäßen Effektoren der DP IV- bzw. der DP IV-analogen Enzymaktivität zur Verhinderung oder Milderung pathologischer Stoffwechsel-Anomalien von Säuger-Organismen wie z.B. beeinträchtigte Glukosetoleranz, Glukosurie, Hyperlipidämie, metabolischen Azidosen, Diabetes mellitus, diabetischer Neuropathie und Nephropathie sowie von durch Diabetes mellitus verursachten Folgeerkrankungen von Säugern. In einer weiteren bevorzugten Ausführungsform betrifft die Erfindung ein Verfahren zur Senkung des Blutzuckerspiegels unter die für Hyperglykämie charakteristische Glukosekonzentration im Serum eines Säuger-Organismus, das dadurch gekennzeichnet ist, daß man einem Säuger-Organismus eine therapeutisch wirksame Menge mindestens eines erfindungsgemäßen Effektors der DP IV- bzw. der DP IV-analogen Enzymaktivität verabreicht.

In einer weiteren bevorzugten Ausführungsform betrifft die Erfindung pharmazeutische Zusammensetzungen, also Medikamente, die mindestens eine erfindungsgemäße Verbindung oder deren Salze gegebenenfalls in Kombination mit einem oder mehreren pharmazeutisch akzeptablen Trägern und/oder Lösungsmitteln enthalten.

Die pharmazeutischen Zusammensetzungen können z.B. als parenterale oder enterale Formulierungen vorliegen und entsprechende Träger enthalten bzw. sie können als orale Formulierungen vorliegen, die entsprechende zur oralen Verabreichung geeignete Träger enthalten können. Vorzugsweise liegen sie als orale Formulierungen vor.

Zusätzlich können die pharmazeutischen Zusammensetzungen einen oder mehrere hypoglykämisch wirkende Wirkstoffe enthalten, die an sich bekannte Wirkstoffe sein können.

Die erfindungsgemäßen Effektoren der DP IV bzw. der DP IV-analogen Enzymaktivität können zur Senkung des Blutzucker-Spiegels unter die für Hyperglykaemie charakteristische Glucose-Konzentration im Serum eines Säuger-Organismus bzw. zur Herstellung eines entsprechenden Medikaments verwendet werden.

Die erfindungsgemäß applizierten Effektoren der DP IV- bzw. DP IV-analoger Enzyme können in pharmazeutisch anwendbaren Formulierungen oder Formulierungskomplexen als Inhibitoren, Substrate, Pseudosubstrate, Inhibitoren der DP IV-Expression, Bindungsproteine oder Antikörper dieser Enzymproteine oder Kombinationen aus diesen verschiedenen Stoffen, die DP IV- bzw. DP IV-analoge Proteinkonzentration im Säugerorganismus reduzieren, zum Einsatz kommen. Effektoren sind z.B. DP IV-Inhibitoren wie die Dipeptidderivate bzw. Dipeptidmimetika die in Tabelle 1 angegebenen Effektoren und deren Fumarsalze. Die erfindungsgemäßen Effektoren ermöglichen eine individuell einstellbare Behandlung von Patienten bzw. Krankheiten, wobei insbesondere individuell auftretende Unverträglichkeiten, Allergien und Nebenwirkungen vermieden werden können.

Auch weisen die Verbindungen unterschiedliche zeitliche Verläufe der Wirksamkeit auf. Dadurch wird dem behandelnden Arzt die Möglichkeit in die Hand gegeben, differenziert auf die individuelle Situation eines Patienten zu reagieren: Einerseits kann er die Geschwindigkeit des Eintritts der Wirkung und andererseits die Dauer der Wirkung und insbesondere die Stärke der Wirkung genau einstellen.

Das erfindungsgemäße Verfahren stellt eine neuartige Herangehensweise zur Senkung erhöhter Blutglukosekonzentration im Serum von Säugern dar. Es ist einfach, kommerziell nutzbar und zur Anwendung bei der Therapie, insbesondere von Erkrankungen, die auf überdurchschnittlichen Blutglukosewerten basieren, bei Säugern und insbesondere in der Humanmedizin geeignet.

Die Effektoren werden z.B. in Form von pharmazeutischen Präparaten verabreicht, die den Wirkstoff in Kombination mit üblichen aus dem Stand der Technik bekannten Trägermaterialien enthalten. Beispielsweise werden sie parenteral (z.B. *i.v.*, in physiologischer Kochsalzlösung) oder enteral (z.B. oral, formuliert mit üblichen Trägermaterialien wie z. B. Glukose) appliziert.

In Abhängigkeit von ihrer endogenen Stabilität und ihrer Bioverfügbarkeit müssen pro Tag einfache oder auch mehrfache Gaben der Effektoren erfolgen, um die erwünschte Normalisierung der Blutglukosewerte zu erreichen. Z. B. kann ein solcher Dosisbereich beim Menschen im Bereich von 0.01 mg bis 30.0 mg pro Tag, vorzugsweise im Bereich von 0.01 bis 10 mg Effektorsubstanz pro Kilogramm Körpergewicht liegen.

Es wurde gefunden, daß durch Verabreichung von Effektoren der Dipeptidyl Peptidase IV bzw. DP IV-analoger Enzymaktivitäten im Blut eines Säugers, durch deren damit verbundene, temporäre Aktivitätsreduktion, in kausaler Folge die endogenen (oder zusätzlich exogen verabreichten) insulinotropen Peptide Gastric Inhibitory Polypeptide 1-42 ($GIP_{1-42}$) und Glucagon-Like Peptide Amide-1 7-36 ($GLP-1_{7-36}$) (o.a. $GLP-1_{7-37}$ oder deren Analoga) durch DP IV- und DP IV-ähnliche Enzyme vermindert abgebaut werden und damit die Konzentrationsabnahme dieser Peptidhormone bzw. ihrer Analoga verringert bzw. verzögert werden. Die durch die Wirkung von DP IV-Effektoren erzielte, erhöhte Stabilität der (endogen vorhandenen oder exogen zugeführten) Incretine oder ihrer Analoga, die damit vermehrt für die insulinotrope Stimulierung der Incretin-Rezeptoren der Langerhansschen Zellen im Pankreas zur Verfügung stehen, verändert u.a. die Wirksamkeit von körpereigenem Insulin, was eine Stimulierung des Kohlehydratstoffwechsels des behandelten Organismus nach sich zieht.

Als Resultat sinkt der Blutzuckerspiegel unter die für Hyperglykämie charakteristische Glukosekonzentration im Serum des behandelten Organismus. Damit können Stoffwechselanomalien wie beeinträchtigte Glukosetoleranz, Glukosurie, Hyperlipidämie sowie mögliche schwere metabolische Azidosen und Diabetes mellitus, Krankheitsbilder die Folge einer über einen längeren Zeitraum erhöhten Glukosekonzentrationen im Blut sind, verhindert bzw. gemildert werden.

In der Reihe der aus dem Stand der Technik bekannten, oral wirksamen Antidiabetika ist bisher eine derartig wirksame, niedermolekulare Substanzklasse (mit Ausnahme des Biguanides Metformin: Molekulargewicht 130) nicht bekannt. Die Molekulargewichte der Aminoacyl Thiazolidide bewegen sich zwischen 146 (Glycyl Thiazolidid), 203 (Isoleucyl Thiazolidid) und 275 (Tryptophanoyl Thiazolidid). Im Vergleich bewegen sich die Molekulargewichte der Sulphonylharnstoffe (Glibenclamid: 494), der Saccharide (Acarbose: 630) sowie der Thiazolidindione (Pioglitazon: 586) im Bereich um 500 bis 700 Da. Physiologisch werden Aminoacyl Thiazolidide durch Aminopeptidasen sowie durch saure Hydrolyse in körpereigene Substanzen, wie Aminosäuren und Cysteamin, hydrolysiert, so daß die Verwendung der erfindungsgemäßen Verbindungen als oral verfügbare Antidiabetika eine Bereicherung der Pharmazie darstellt.

Bei Ratten und Mäusen ist experimentell induzierte Hyperglykämie durch orale Verabreichung mit den erfindungsgemäß verwendeten Verbindungen überdurchschnittlich gut behandelbar (Tabellen 2 und 3). Die Verabreichung des 500- bis 1000-fachen der wirksamen Dosis führte zu keiner nachweisbaren pathologischen Veränderung während drei-wöchiger toxikologischer Experimente an Ratten und Mäusen.

Die vorteilhafte Wirkung von Verbindungen auf DP IV ist in Tabelle 1 beispielhaft belegt:

Tabelle 1:

| Wirkung verschiedener Effektoren auf die durch Dipeptidyl Peptidase IV katalysierte Hydrolyse von 0,4 mM des Substrates H-Gly-Pro-pNA bei 30°C, pH 7,6 und einer Ionenstärke von 0,125. | | |
|---|---|---|
| **Effektor** | **Effektoraffinität zu DP IV: $K_i$ [nM]** | **% Restaktivität der DP IV in Gegenwart von 10 $\mu$M Effektor** |
| Metformin | » 1.000.000 | 100 |
| Glibenclamid | » 1.000.000 | 100 |
| Acarbose | » 1.000.000 | 100 |
| H-Asn-Pyrrolidid | 12.000 | 83,1 |
| H-Asn-Thiazolidid | 3.500 | 47,2 |
| H-Asp-Pyrrolidid | 14.000 | 81,6 |
| H-Asp-Thiazolidid | 2.900 | 45,6 |
| H-Asp(NHOH)-Pyrrolidid | 13.000 | 88,2 |
| H-Asp(NHOH)-Thiazolidid | 8.800 | 54,5 |
| H-Glu-Pyrrolidid | 2.200 | 38,5 |
| H-Glu-Thiazolidid | 610 | 25,0 |
| H-Glu(NHOH)-Pyrrolidid | 2.800 | 44,9 |
| H-Glu(NHOH)-Thiazolidid | 1.700 | 36,5 |
| H-His-Pyrrolidid | 3.500 | 49,7 |
| H-His-Thiazolidid | 1.800 | 35,2 |
| H-Pro-Pvrrolidid | 4.100 | 50,2 |
| H-Pro-Thiazolidid | 1.200 | 27,2 |
| H-Ile-Azididid | 3.100 | 43,8 |
| H-Ile-Pyrrolidid | 210 | 12,3 |
| **H-L-allo-Ile-Thiazolidid** | **190** | **10,0** |
| H-Val-Pyrrolidid | 480 | 23,3 |
| H-Val-Thiazolidid | 270 | 13,6 |

Es ist bekannt, daß Aminoacyl Pyrrolidide und Aminoacyl Thiazolidide durch die in den Mucosazellen des Dünndarms, im Serum und in Leberzellen vorhandenen Enzyme Prolin Aminopeptidase und Prolidase abgebaut werden können und der Thiazolidinring zur Öffnung in Gegenwart von Säuren (beispielsweise im Magen) unter Bildung des adäquaten Cysteamin-Derivates neigt [vgl. US 458407]. Es war daher überraschend, eine dosisabhängige Wirksamkeit der Wirkstoffe nach per oraler Verabreichung zu finden. Die Dosisabhängigkeit der Wirkung von L-allo-Ile-Thiazolidid auf die Serum-DP IV Aktivität nach oraler Applikation von L-allo-Isoleucyl Thiazolidid an gesunden Wistarratten ist mit folgender Tabelle belegt:

Tabelle 2:

| Restaktivität der DP IV im Serum gegenüber 0,4 mM des Substrates H-Gly-Pro-pNA bei 30°C, pH 7,6 und einer Ionenstärke von 0,125, nach oraler Gabe und in Abhängigkeit von der Dosis L-allo-Isoleucyl Thiazolidid, bestimmt 30 min nach Applikation des Inhibitors. | |
|---|---|
| Dosis pro Versuchstier | Restaktivität der DP IV in % |
| 0 mg | 100 |
| 2,5 mg | 52 |

Tabelle 2: (fortgesetzt)

| Restaktivität der DP IV im Serum gegenüber 0,4 mM des Substrates H-Gly-Pro-pNA bei 30°C, pH 7,6 und einer Ionenstärke von 0,125, nach oraler Gabe und in Abhängigkeit von der Dosis L-allo-Isoleucyl Thiazolidid, bestimmt 30 min nach Applikation des Inhibitors. | |
| --- | --- |
| Dosis pro Versuchstier | Restaktivität der DP IV in % |
| 5,0 mg | 40 |
| 10 mg | 28 |
| 20 mg | 29 |

Ausgesprochen überraschend und wünschenswert ist die im diabetischen Tiermodell erzielte Glukose-reduzierende Wirkung des erfindungsgemäßen Wirkstoffs L-allo-Isoleucyl Thiazolidid nach seiner oralen Verabreichung bei zeitgleicher oraler Glukose-Stimulierung (Tabelle 3).

Zur Verstärkung der blutzuckersenkenden Wirkung verschiedener Antidiabetika werden häufig Kombinationen verschiedener oral wirksamer Antidiabetika eingesetzt. Da sich die antihyperglykämische Wirkung der efindungsgemäßen Effektoren unabhängig von anderen bekannten oral applizierbaren Antidiabetika entfaltet, eignen sich die erfindungsgemäßen Wirkstoffe analog in entsprechender galenischer Form, zur Erzielung des gewünschten normoglykämischen Effektes, zum Einsatz bei Kombinationstherapien.

Somit können die erfindungsgemäß verwendeten Verbindungen in an sich bekannter Weise in die üblichen Formulierungen überführt werden, wie z. B. Tabletten, Kapseln, Dragees, Pillen, Suppositorien, Granulate, Aerosole, Sirupe, flüssige, feste und cremeartige Emulsionen und Suspensionen und Lösungen unter Verwendung inerter, untoxischer, pharmazeutisch geeigneter Träger- und Zusatzstoffe oder Lösungsmittel. Hierbei liegen die therapeutisch wirksamen Verbindungen jeweils vorzugsweise in einer Konzentration von etwa 0.1 bis 80, vorzugsweise von 1 bis 50 Masseprozent der Gesamtmischung vor, d.h. in Mengen, die ausreichend sind, um den angegebenen Dosierungsspielraum zu erreichen.

Tabelle 3:

| Reduktion der zirkulierenden Blutglukose innerhalb 60 min nach oraler Verabreichung von 20 $\mu$M L-allo-Ile-Thiazolidid an Ratten verschiedener Tiermodelle bei zeitgleichem Glukosetoleranztest (Angaben in % bezogen auf normoglykämische Werte). | | |
| --- | --- | --- |
| Tiermodell | Glukosekonzentration in % Kontrolle | Glukosekonzentration in % L-allo-Ile-Thiazolidid behandelt |
| Wistarratte, normal | 100 | 82 |
| Wistarratte (Diabetes 2b -Model, dick) | 100 | 73 |

Die gute Resorption der erfindungsgemäß verwendeten Verbindungen durch Schleimhäute des gastrointestinalen Traktes ermöglicht die Anwendung von vielen galenischen Zubereitungen:

Die Substanzen können als Medikament in Form von Dragees, Kapseln, Beißkapseln, Tabletten, Tropfen, Sirup, aber auch als Zäpfchen oder als Nasensprays angewendet werden.

Die Formulierungen werden beispielsweise hergestellt durch Strecken des Wirkstoffs mit Lösungsmitteln und/oder Trägerstoffen, gegebenenfalls unter Verwendung von Emulgatoren und/oder Dispergiermitteln, wobei z. B. im Fall des Einsatzes von Wasser als Verdünnungsmittel gegebenenfalls organische Lösungsmittel als Hilfslösungsmittel verwendet werden können.

Als Hilfsstoffe seien beispielhaft aufgeführt: Wasser, untoxische organische Lösungsmittel, wie Paraffine (z. B. Erdölfraktionen), pflanzliche Öle (z. B. Rapsöl, Erdnußöl, Sesamöl), Alkohole (z.B. Ethylalkohol, Glycerin), Glykole (z.B. Propylenglykol, Polyetylenglykol); feste Trägerstoffe, wie z. B. natürliche Gesteinsmehle (z. B. hochdisperse Kieselsäure, Silikate), Zucker (z.B. Roh-, Milch- und Traubenzucker); Emulgiermittel, wie nichtionogene und anionische Emulgatoren (z. B. Polyoxyethylen-Fettsäure-Ester, Polyoxyethylen-Fettalkohol-Ether, Alkylsulfonate und Arylsulfonate), Dispergiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat) und gegebenenfalls Aromastoffe.

Die Applikation erfolgt in üblicher Weise, vorzugsweise enteral oder parenteral, insbesondere oral. Im Falle der enteralen Anwendung können Tabletten außer den genannten Trägerstoffen weitere Zusätze wie Natriumcitrat, Calciumcarbonat und Calciumphosphat, zusammen mit verschiedenen Zuschlagstoffen, wie Stärke, vorzugsweise

Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen und/oder Elixieren, die für orale Anwendungen gedacht sind, können die Wirkstoffe außer mit den oben genannten Hilfsstoffen zusätzlich mit verschiedenen Geschmacksaufbesserern oder Farbstoffen versetzt werden.

Bei einer parenteralen Anwendung können Lösungen der Wirkstoffe unter Verwendung geeigneter flüssiger Trägermaterialien eingesetzt werden. Im allgemeinen hat es sich als vorteilhaft erwiesen, bei intravenöser Applikation Mengen von etwa 0,01 bis 2,0 mg/kg, vorzugsweise etwa 0,01 bis 1,0 mg/kg Körpergewicht pro Tag zur Erreichung wirksamer Ergebnisse zu verabreichen, und bei enteraler Applikation beträgt die Dosierung etwa 0,01 bis 2 mg/kg, vorzugsweise etwa 0,01 bis 1 mg/kg Körpergewicht pro Tag.

Trotzdem kann es gegebenenfalls erforderlich sein, von den genannten Mengen abzuweichen, und zwar in Abhängigkeit vom Körpergewicht des Versuchstieres oder Patienten bzw. der Art des Applikationsweges, aber auch aufgrund der Tierart und deren individuellem Verhalten gegenüber dem Medikament bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Fall der Applikation größer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäß gelten hierbei auch die obigen Ausführungen.

**Beispiele für pharmazeutische Formulierungen**

1. Kapseln mit 100 mg L-allo-Isoleucyl Thiazolidid pro Kapsel (nicht Teil der Erfindung)

Für ca. 10 000 Kapseln wird eine Lösung folgender Zusammensetzung hergestellt:

| | |
|---|---|
| L-allo-Isoleucyl Thiazolidid Hydrochlorid | 1,0 kg |
| Glycerin | 0,5 kg |
| Polyethylenglykol | 3,0 kg |
| Wasser | 0,5 kg |
| | 5,0 kg |

Die Lösung wird nach an sich bekannter weise in weichgelatinekapseln abgefüllt. Die Kapseln sind zum Zerbeißen oder zum Schlucken geeignet.

2. Tabletten bzw. lackierte Tabletten oder Dragees mit 100 mg L-allo-Isoleucyl Thiazolidid (nicht Teil der Erfindung)

Die folgenden Mengen beziehen sich auf Herstellung von 100 000 Tabletten:

| | |
|---|---|
| L-allo-Isoleucyl Thiazolidid Hydrochlorid, fein vermahlen | 10,0 Kg |
| Glukose | 4,35 Kg |
| Milchzucker | 4,35 Kg |
| Stärke | 4,50 Kg |
| Zellulose, fein vermahlen | 4,50 Kg |

Obige Bestandteile werden gemischt und anschließend mit einer Lösung, hergestellt aus

| | |
|---|---|
| Polyvinylpyrrolidon | 2,0 kg |
| Polysorbat | 0,1 kg |
| und Wasser | ca. 5,0 kg |

versehen und in an sich bekannter Weise granuliert, indem die feuchte Masse geraspelt und nach Zugabe von 0,2 kg Magnesiumstearat getrocknet wird. Die fertige Tablettenmischung von 30,0 kg wird zu gewölbten Tabletten von 300 mg Gewicht verarbeitet. Die Tabletten können nach an sich bekannter Weise lackiert oder dragiert werden.

Die technischen Daten bevorzugter Verbindungen werden nachstehend angegeben.

| Untersuchungen zu Ile-Thia*Fumarat (Isomere) und anderen Salzen | | | | | |
|---|---|---|---|---|---|
| Substanz | $K_i$ | Fp (°C) | CE (min) | MS | $[\alpha]H_2O$ |
| L-threo-IT*F | $8*10^{-8}$ | $150^{DSC}$ | 160 | 203 | -10,7 (405nm) |
| D-threo-IT*F | keine Hemmung | 147 | 158 | 203 | nicht bestimmt |
| L-allo-IT*F nicht Teil der Erfindung | $2*10^{-7}$ | 145-6 | 154 | 203 | -4,58 (380nm) |
| D-allo-IT*F nicht Teil der Erfindung | keine Hemmung | 144-6 | 150 | 203 | 4,5 (380nm) |
| IT*F = Isoleucyl-Thiazolidid Fumarat Die NMR und HPLC Daten bestätigen, daß es sich um die entsprechenden Substanzen handelte | | | | | |

| Meßbedingungen für die $K_i$-Bestimmung der Substanzen | |
|---|---|
| Enzym | DPIV$_{Schweineniere}$, 0,75mg/ml, 18U/ml (GPpNA) in 25mM Tris pH 7,6,30% Ammoniumsulfat, 0,5mM EDTA, 0,5mM DTE *Stammlösung:* 1:250 verdünnt in Meßpuffer |
| Puffer | 40mM HEPES pH 7,6, I=0,125 (KCl) |
| Substrat | GPpNA*HCl *Stammlösung:* 2,1mM |
| Meßgerät | Perkin-Elmer Bio Assay Reader, HTS 7000 Plus, T=30°C λ=405nm |
| Meßansatz | 100µl Puffer 100µl Substrat (3 verschiedene Konzentrationen 0,8mM - 0,2mM) 50µl Wasser/Inhibitor (7 verschiedene Konzentrationen 2,1µM - 32,8nM) 10µl Enzym |

Puffer, Wasser/Inhibitor und Enzym wurden auf 30°C vortemperiert und die Reaktion durch die Zugabe von ebenfalls vortemperiertem Substrat gestartet.
Es wurden 4fach Bestimmungen durchgeführt.
Die Meßzeit betrug 10min.

**Schmelzpunktbestimmung**

Schmelzpunkte wurden an einem Kofler-Heiztischmikroskop der Leica-Aktiengesellschaft, die Werte sind nicht korrigiert, oder an einem DSC-Gerät (bei Heumann-Pharma) bestimmt.

**Optische Rotation**

Die Drehwerte wurden bei unterschiedlichen Wellenlängen an einem "Polarimeter 341" oder höher der Fa. Perkin Elmer aufgenommen.

**Meßbedingungen für die Massenspektrometrie**

Die Massenspektren wurden an einem "API 165" bzw. "API 365" der Fa. PE Sciex mittels Elektrosprayionisation (ESI) aufgenommen.
Es wird mit einer ungefähren Konzentration von c = 10 µg/ml gearbeitet, die Substanz wird in MeOH / $H_2O$ 50:50, 0,1 % $HCO_2H$ aufgenommen, die Infusion erfolgt mit Spritzenpumpe (20µl/min).Die Messungen erfolgten im Positivmodus $[M+H]^+$, die ESI-Spannung beträgt U = 5600V.
Die Salze weisen die folgenden Daten auf:

| IT*Salz | $K_i$ | M (gmol$^{-1}$) | Fp (°C) |
|---|---|---|---|
| Succinat | 5,1 e-8 | 522,73 | 116 |
| Tartrat | 8,3 e-8 | 352,41 | 122 |
| Fumarat | 8,3 e-8 | 520,71 | 156 |

**Löslichkeitsuntersuchung der Salze des Ile-Thia**

Ile-Thia*Fum

Einwaage 10,55 mg
entspricht 0,02 mmol (520,72 g/mol)
Zugabe von 100 µl $H_2O_{dest}$
100 µl keine Lösung, optisch: keine Oberflächenbenetzung
ab 200 µl sukzessiver Beginn der Löslichkeit
bei 400µl ist eine vollständiges Lösen zu beobachten 2,63 %

Bei diesem Salz wurde also festgestellt, daß es kaum benetzbar ist und sich nicht zersetzt.

Ile-Thia*Succ

Einwaage 16,6 mg
entspricht 0,031 mmol (522,73 g/mol)
Zugabe von 16 µl $H_2O_{dest}$
16 µl keine Lösung, optisch "Aufsaugen" der Feuchtigkeit
von 66 µl.- 1,5 ml kein vollständiges Lösen der Substanz zu beobachten

Ile-Thia*Tartrat

Einwaage 17,3 mg
entspricht 0,049 mmol (352,41 g/mol)
Zugabe von 100 µl $H_2O_{dest}$
100 µl vollständiges Lösen
17,3 %

**Allgemeine Synthese Ile-Thia*Salz**

Die Boc-geschüzte Aminosäure Boc-Ile-OH wird in Essigsäureethylester vorgelegt, der Ansatz wird auf ca. -5 °C gekühlt. N-Methylmorpholin wird zugetropft, Pivalinsäurechlorid (Labor) bzw. Neohexanoylchlorid (Technikum) wird unter Temperaturkonstanz zugetropft. Die Reaktion wird für wenige Minuten zur Aktivierung gerührt. N-Methylmorpholin (Labor) und Thiazolidinhydrochlorid (Labor) werde nacheinander zugetropft, Thiazolidin (Technikum) wird zugegeben. Die Aufarbeitung im Labor erfolgt klassisch mit Salzlösungen, im Technikum wird der Ansatz mit NaOH- und $CH_3COOH$-Lösungen gereinigt.
Die Abspaltung der Boc-Schutzgruppe wird durch HCl / Dioxan (Labor) bzw. $H_2SO_4$ (Technikum) erzielt.
Im Labor wird das Hydrochlorid aus EtOH / Ether kristallisiert.
Im Technikum wird das freie Amin durch Zugabe von NaOH / $NH_3$ dargestellt. Fumarsäure wird in heißen Ethanol gelöst, das freie Amin wird zugetropft, es fällt (Ile-Thia)$_2$-Fumarat (M = 520,71 gmol$^{-1}$).
Die Analytik von Isomeren bzw. Enantiomeren erfolgt durch Elektrophorese.

**EP 1 215 207 B1**

**Patentansprüche**

1. Organische Salze von Dipeptidmimetika, die aus einer Aminosäure und einer Thiazolidin- oder Pyrrolidingruppe gebildet werden, nämlich organische Salze von L-threo-Isoleucyl Thiazolidin und von L-threo-Isoleucyl Pyrrolidin.

2. Salze nach Anspruch 1, nämlich die Succinate, Tartrate, und Fumarate.

3. Pharmazeutische Zusammensetzung, **dadurch gekennzeichnet, daß** sie mindestens eine Verbindung nach einem der vorstehenden Ansprüche gegebenenfalls in Kombination mit einem oder mehreren pharmazeutisch akzeptablen Trägern und/oder Lösungsmitteln enthält.

4. Pharmazeutische Zusammensetzung nach Anspruch 3, **dadurch gekennzeichnet, daß** es sich um einen Träger für parenterale oder enterale Formulierungen handelt.

5. Pharmazeutische Zusammensetzung nach Anspurch 3, **dadurch gekennzeichnet, daß** sie in einer Formulierung zur oralen Verabreichung vorliegt.

6. Pharmazeutische Zusammensetzung nach einem der Ansprüche 3 bis 5, **dadurch gekennzeichnet, daß** sie zusätzlich einen hypoglykämisch wirkenden Wirkstoff enthält.

7. Verwendung von mindestens einer Verbindung oder pharmazeutischen Zusammensetzung nach einem der vorstehenden Ansprüche zur Herstellung eines Medikamentes zur Aktivitätsminderung von Dipeptidylpeptidase IV bzw. von Dipeptidylpeptidase IV analogen Enzymaktivitäten.

8. Verwendung von mindestens einer Verbindung oder Zusammensetzung nach einem der Ansprüche 1 bis 6 zur Herstellung eines Medikamentes zur Senkung des Blutzuckerspiegels unter die für Hyperglykämie charakteristische Glukosekonzentration im Serum eines Säugers.

9. Verwendung von mindestens einer Verbindung oder Zusammensetzung nach einem der Ansprüche 1 bis 6 zur Herstellung eines Medikamentes zur oralen Behandlung von mit Diabetes mellitus im Zusammenhang stehenden Stoffwechselerkrankungen.

10. Verwendung von mindestens einer Verbindung oder Zusammensetzung nach einem der Ansprüche 1 bis 6 zur Herstellung eines Medikamentes zur Behandlung von beeinträchtigter Glukosetoleranz, Glukosurie, Hyperlipidämie, metabolischen Azidosen, Diabetes mellitus, diabetischer Neuropathie und Nephropathie sowie von durch Diabetes mellitus verursachten Folgeerkrankungen von Säugern.

**Claims**

1. Organic salts of dipeptide mimetics formed from an amino acid and a thiazolidine or pyrrolidine group namely organic salts of L-threo-isoleucyl thiazolidine and L-threo-isoleucyl pyrrolidine.

2. Salts according to claim 1, namely succinates, tartrates and fumarates.

3. Pharmaceutical composition, **characterised in that** it comprises at least one compound according to any one of the preceding claims optionally in combination with one or more pharmaceutically acceptable carriers and/or solvents.

4. Pharmaceutical composition according to claim 3, **characterised in that** the carrier is a carrier for parenteral or enteral formulations.

5. Pharmaceutical composition according to claim 3, **characterised in that** it is present in a formulation for oral administration.

6. Pharmaceutical composition according to any one of claims 3 to 5, **characterised in that** it additionally comprises an active ingredient having hypoglycaemic action.

7. Use of at least one compound or pharmaceutical composition according to any one of the preceding claims in the production of a medicament for reducing the activity of dipeptidyl peptidase IV or of dipeptidyl peptidase IV-analogous enzyme activities.

8. Use of at least one compound or composition according to any one of claims 1 to 6 in the production of a medicament for lowering the blood sugar level below the glucose concentration that is characteristic of hyperglycaemia in the serum of a mammal.

9. Use of at least one compound or composition according to any one of claims 1 to 6 in the production of a medicament for the oral treatment of metabolic disorders associated with diabetes mellitus.

10. Use of at least one compound or composition according to any one of claims 1 to 6 in the production of a medicament for the treatment of impaired glucose tolerance, glycosuria, hyperlipidaemia, metabolic acidoses, diabetes mellitus, diabetic neuropathy and nephropathy and also of sequelae of diabetes mellitus in mammals.

**Revendications**

1. Sels organiques de substances mimétiques dipeptidiques, qui peuvent être formés à partir d'un acide aminé et d'un groupe thiazolidine ou pyrrolidine, à savoir des sels organiques de L-thréo-isoleucyl-thiazolidine et de L-thréo-isoleucyl-pyrrolidine.

2. Sels selon la revendication 1, à savoir les succinates, les tartrates et les fumarates.

3. Composition pharmaceutique, **caractérisée en ce qu'**elle contient au moins un composé selon l'une des revendications précédentes combiné le cas échéant à un ou plusieurs véhicules pharmaceutiquement acceptables et/ou à des solvants.

4. Composition pharmaceutique selon la revendication 3, **caractérisée en ce qu'**il s'agit d'un véhicule pour des formulations à administration parentérale ou entérale.

5. Composition pharmaceutique selon la revendication 3, **caractérisée en ce qu'**elle existe dans une formulation destinée à une administration orale.

6. Composition pharmaceutique selon l'une des revendications 3 à 5, **caractérisée en ce qu'**elle contient en outre une substance hypoglycémiante.

7. Utilisation d'au moins un composé ou d'une composition pharmaceutique selon l'une des revendications précédentes, pour la fabrication d'un médicament destiné à réduire l'activité de la dipeptidylpeptidase IV, le cas échéant les activités enzymatiques analogues à la dipeptidylpeptidase IV.

8. Utilisation d'au moins un composé ou une composition selon l'une des revendications 1 à 6, pour la fabrication d'un médicament destiné à faire baisser le taux de sucre dans le sang sous la concentration de glucose caractéristique de l'hyperglycémie dans le sérum d'un mammifère.

9. Utilisation d'au moins un composé ou une composition selon l'une des revendications 1 à 6, pour la fabrication d'un médicament destiné au traitement oral de pathologies métaboliques associées au diabète sucré.

10. Utilisation d'au moins un composé ou une composition selon l'une des revendications 1 à 6, pour la fabrication d'un médicament destiné au traitement de la tolérance au glucose altérée, de la glycosurie, de l'hyperlipidémie, des acidoses métaboliques, du diabète sucré, de la neuropathie et de la néphropathie diabétiques ainsi que des pathologies secondaires des mammifères provoquées par le diabète sucré.

Kapillarzonenelektrophorese (CE) -Trennung der Isomere des Isoleucyl Thiazolidids

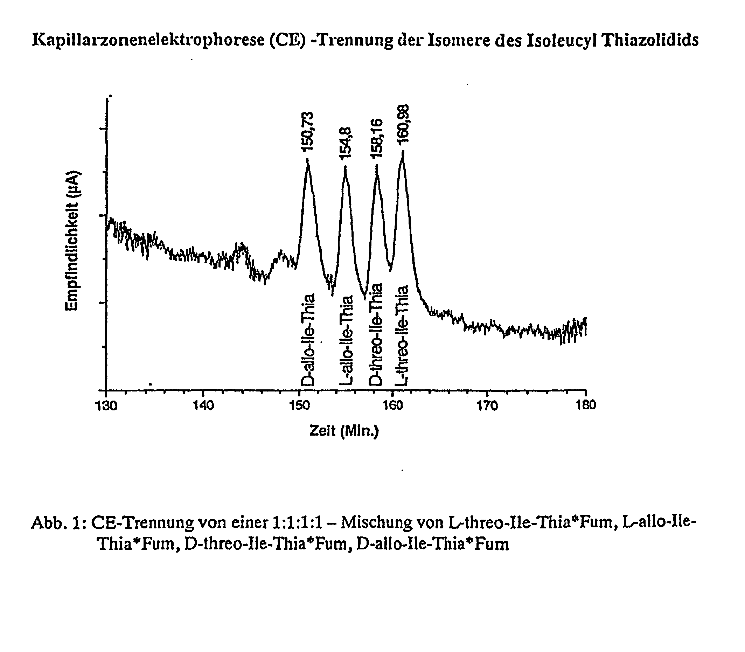

Abb. 1: CE-Trennung von einer 1:1:1:1 – Mischung von L-threo-Ile-Thia*Fum, L-allo-Ile-Thia*Fum, D-threo-Ile-Thia*Fum, D-allo-Ile-Thia*Fum

Methode und Meßbedingungen:

CE-Untersuchungen erfolgten am „P/ACE™ System MDQ" der Firma Beckman:

| Substanz | CE (min) |
|----------|----------|
| L-threo-IT*F | 160 |
| D-threo-IT*F | 158 |
| L-allo-IT*F | 154 |
| D-allo-IT*F | 150 |

Laufbedingungen:

Puffer:      20 mM Phosphat, pH 7,0, 100 mM β-Hydroxy-propyl-cyclodextrin
Kapillare:   50 / 60,2 cm, 25 μm Innendurchmesser, beschichtet mit Acrylamid
Spannung:    10 kV
Detektion:   Photo-Dioden-Array-Detektor bei 214 nm
Temperatur:  7°C

**CE-Trennung von Ile-Thia*Fumarat**

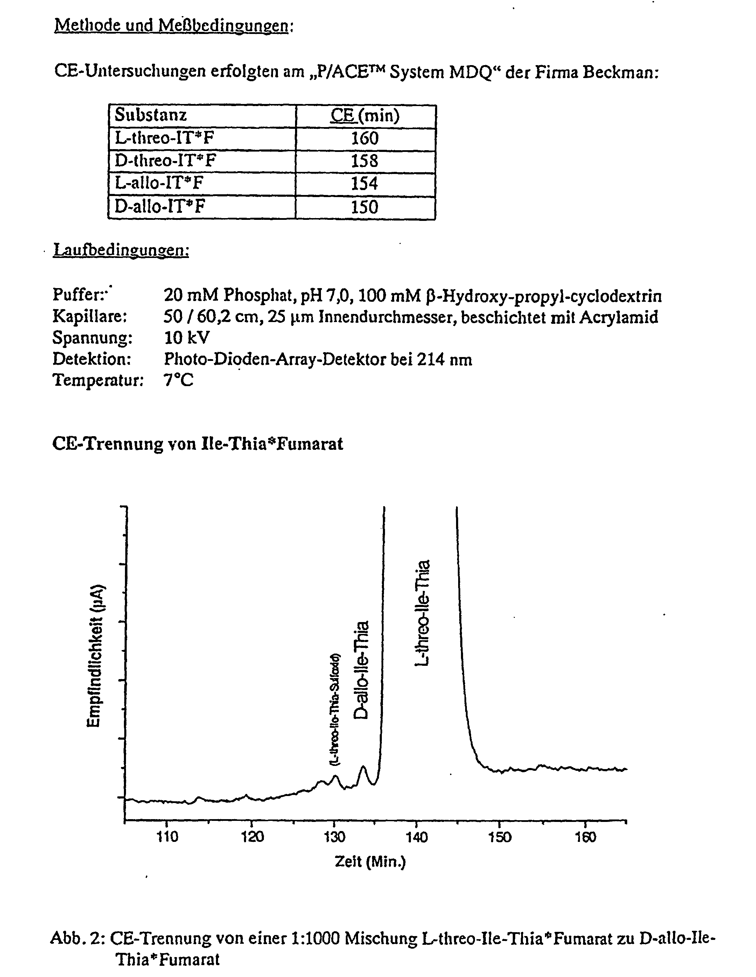

Abb. 2: CE-Trennung von einer 1:1000 Mischung L-threo-Ile-Thia*Fumarat zu D-allo-Ile-Thia*Fumarat

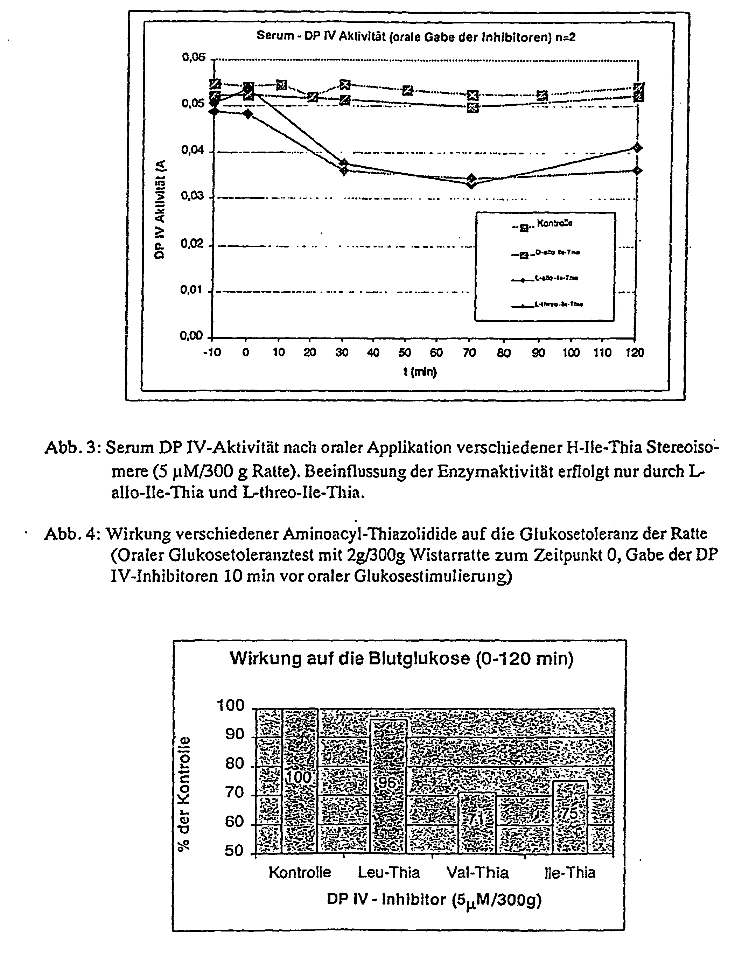

Abb. 3: Serum DP IV-Aktivität nach oraler Applikation verschiedener H-Ile-Thia Stereoisomere (5 µM/300 g Ratte). Beeinflussung der Enzymaktivität erfolgt nur durch L-allo-Ile-Thia und L-threo-Ile-Thia.

Abb. 4: Wirkung verschiedener Aminoacyl-Thiazolidide auf die Glukosetoleranz der Ratte (Oraler Glukosetoleranztest mit 2g/300g Wistarratte zum Zeitpunkt 0, Gabe der DP IV-Inhibitoren 10 min vor oraler Glukosestimulierung)